(19) Europäisches Patentamt
European Patent Office
Office européen des brevets

(11) Veröffentlichungsnummer: **0 097 935**
**B1**

(12) # EUROPÄISCHE PATENTSCHRIFT

(45) Veröffentlichungstag der Patentschrift: **20.07.88**

(51) Int. Cl.⁴: **B 01 J 23/66,** B 01 J 37/02, C 07 D 301/10

(21) Anmeldenummer: **83106212.0**

(22) Anmeldetag: **25.06.83**

(54) **Silberkatalysatoren, Verfahren zu ihrer Herstellung und ihre Verwendung zur Herstellung von Ethylenoxid.**

(30) Priorität: **30.06.82 DE 3224322**
**30.06.82 DE 3224323**

(43) Veröffentlichungstag der Anmeldung: **11.01.84 Patentblatt 84/2**

(45) Bekanntmachung des Hinweises auf die Patenterteilung: **20.07.88 Patentblatt 88/29**

(84) Benannte Vertragsstaaten: **BE DE FR GB IT NL**

(56) Entgegenhaltungen:
**EP-A-0 025 963**
**DE-A-2 448 449**
**DE-A-2 733 688**
**GB-A-2 002 252**
**US-A-4 305 844**

(73) Patentinhaber: **HOECHST AKTIENGESELLSCHAFT, Postfach 80 03 20, D-6230 Frankfurt am Main 80 (DE)**

(72) Erfinder: **Rebsdat, Siegfried, Dr., Trenbeckstrasse 24, D-8261 Burg, Neuötting (DE)**
Erfinder: **Mayer, Sigmund, Hochfellnstrasse 20, D-8261 Burgkirchen/Alz (DE)**
Erfinder: **Alfranseder, Josef, Hauptstrasse 31, D-8261 Hofschallern Post Marktl (DE)**

EP 0 097 935 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft Silberkatalysatoren, die aus Silber und Promotor auf einem hitzebeständigen, porösen Trägermaterial bestehen. Sie betrifft ferner ein Verfahren zu Herstellung dieser Katalysatoren und ihre Verwendung zur Herstellung von Ethylenoxid durch Oxidation von Ethylen mit Sauerstoff.

Ethylenoxid wird großtechnisch durch Direktoxidation von Ethylen mit Sauerstoff über einem Silberkatalysator hergestellt. Eine allgemeine Beschreibung des Verfahrens findet sich in Kirk-Othmer: Encyclopedia of Chemical Technology, Band 9, Seiten 432 bis 471, John Wiley, London-NY, 1980.

Ein ethylen- und sauerstoffhaltiges Gas tritt ober in den Reaktor ein. Dieser besteht aus einem Bündel von mehreren tausend Rohren von 6 bis über 10 m Länge. Das Gas strömt über den in den Rohren befindlichen Katalysator, vorzugsweise bei einer Temperatur von 200 bis 300°C und 1 bis 2 MPa Überdruck, wobei sich 5 bis 20 % des eingesetzten Ethylens umsetzen.

Neben der Bildung von Ethylenoxid wird ein erheblicher Anteil des Ethylens, nämlich etwa 25 %, zu Kohlendioxid und Wasser oxidiert (Totaloxidation), was die nachstehenden Reaktionsgleichungen veranschaulichen sollen:

Ethylenoxidbildung $2C_2H_4 + O_2 \rightarrow 2C_2H_4O$

Totaloxidation $C_2H_4 + 3O_2 \rightarrow 2CO_2 + 2H_2O$.

Zur Temperaturkontrolle und Abfuhr der Wärme befindet sich um die Rohre ein Wärmeträgermedium, das die freiwerdende Wärme aus dem Reaktor heraustransportiert. Das ethylenoxid- und kohlendioxidhaltige Reaktionsgas verläßt den Reaktor, um in den Aufarbeitungsteil zu gelangen, wo Ethylenoxid und Kohlendioxid abgetrennt werden. Das von Ethylenoxid und Kohlendioxid befreite Gas wird erneut mit Ethylen und Sauerstoff angereichert und wieder zum Reaktor gefördert. Es handelt sich um einen kontinuierlichen Kreisprozeß mit heterogener Katalyse.

Als Katalysator dient ein Silberträgerkatalysator. Die Qualität eines derartigen Katalysators wird wesentlich durch seine Selektivität, seine Aktivität und seine Lebensdauer gekennzeichnet.

Die Selektivität ist der molare Prozentsatz an umgesetztem Ethylen, der zu Ethylenoxid reagiert.

Die Aktivität wird durch die Ethylenoxid-Konzentration am Reaktorausgang bei sonst konstanten Bedingungen (z. B. Temperatur, Druck, Gasmenge, Katalysatormenge) charakterisiert. Je höher die Ethylenoxid-Konzentration desto höher die Aktivität. Oder anders ausgedrückt: Je niedriger die zum Erreichen einer bestimmten Ethylenoxid-Konzentration erforderliche Temperetur desto höher die Aktivität.

Neben der Aktivität und Selektivität ist die Lebensdauer des Katalysators ein wichtiges Qualitätsmerkmal. Die Wirksamkeit der Katalysatoren wird mit fortschreitender Einsatzdauer schlechter, das heißt, Aktivität und Selektivität nehmen ständig ab, so daß in Abhängigkeit von der Alterungsgeschwindigkeit früher oder später ein Katalysatorwechsel vorgenommen werden muß. Ein solcher Katalysatortausch ist aber umständlich, teuer und mit Materialverlusten verbunden. Der alte Katalysator muß aus mehreren tausend Reaktorrohren Rohr für Rohr entleert und anschließend der neue Katalysator wieder eingefüllt werden. Dies nimmt einige Wochen in Anspruch, und es entsteht ein entsprechender Produktionsverlust. Der alte Katalysator muß ferner zur Wiedergewinnung des Silbers umgearbeitet werden. Dabei tritt unvermeidlich ein Silber-Verlust auf. Das Trägermaterial kann nicht wiederverwendet werden. Auch bei der Herstellung eines neuen Silberkatalysators treten Silber-Verluste auf. Große Mengen Silber müssen transportiert, versichert, verzinst und eventuell verzollt werden. Ein guter Silberkatalysator für die Herstellung von Ethylenoxid durch Direktoxidation von Ethylen sollte also eine möglichst hohe Aktivität und Selektivität und auch eine hohe Lebensdauer besitzen.

Die Herstellung von Silberträgerkatalysatoren an sich ist schon seit langem bekannt. Sie werden bevorzugt nach dem folgenden Verfahren hergestellt:

Das Trägermaterial wird mit einer Lösung eines Silbersalzes getränkt.

Das getränkte Trägermaterial wird getrocknet, wobei sich das Silbersalz auf dem Trägermaterial niederschlägt. Das so mit Silbersalz imprägnierte Trägermaterial wird Bedingungen unterworfen, unter denen eine Zersetzung des Silbersalzes erfolgt, wobei sich elementares Silber bildet, das auf dem Träger in fein verteilter Form vorliegt. Dieser Schritt kann rein thermisch durch Erhitzen auf etwa 170 bis 400°C oder durch Reduktionsmittel, wie Formaldehyd, erfolgen.

Schon frühzeitig ist erkannt worden, daß die Wirksamkeit der Silberkatalysatoren durch sogenannte Promotoren verbessert werden kann. Als Promotoren werden solche chemische Elemente bezeichnet, die bereits in einer Menge von 10 bis 1000 mg/kg, bezogen auf die Menge an Silber, zu einer Erhöhung der Wirksamkeit beitragen. Die Elemente Kalium, Rubidium und Cäsium haben sich als besonders wirksame Promotoren herausgestellt. Damit lassen sich, bei guter Aktivität, Selektivitäten von etwa 80 % erzielen.

In neuerer Zeit ist festgestellt worden, daß bei der Herstellung von neuen Silberträgerkatalysatoren mit hoher Wirksamkeit nicht nur der Promotor selbst, sondern auch die Art und Weise seines Aufbringens auf das Trägermaterial, nämlich der Zeitpunkt seines Aufbringens in bezug auf das Aufbringen des Silbers,eine wichtige Rolle spielt. Dabei scheint es auch von einiger Bedeutung zu sein, wann die Reduktion der aufgebrachten Silberverbindung zu metallischem Silber erfolgt.

Nach DE-A-2 448 449 soll es vorteilhaft sein, zuerst die gesamte Promotormenge und erst dann die gesamte Silbermenge auf den Träger abzuscheiden, wobei anschließend die Reduktion der abgeschiedenen

Silberverbindung zu metallischem Silber durchgeführt wird. Im Gegensatz dazu wird in DE-A-2 733 688 und 3 011 717 und in GB-B-2 002 252 empfohlen, bei der Herstellung von neuen Silberträgerkatalysatoren mit verbesserter Wirksamkeit in einer ersten Imprägnierung die gesamte Silbermenge und in einer anschließenden zweiten Imprägnierung die gesamte Promotormenge aufzubringen, wobei die Reduzierung der aufgebrachten Silberverbindung nach der ersten Imprägnierung unter teilweise speziellen Bedingungen vorgenommen wird. Neben diesen beiden Vorgangsweisen ist ferner die Methode des gleichzeitigen Aufbringens von Silber und Promotor auf das Trägermaterial bekannt. Diese Art der Herstellung neuer Silberkatalysatoren mit erhöhter Wirksamkeit ist in DE-A-2 300 512, DE-A-2 734 912, DE-A-2 951 969 und DE-A-2 951 970 beschrieben.

Der aus DE-A-2 300 512 bekannte Silberkatalysator besteht aus Silber in einer Menge von 1,5 bis 20 Gew.-% und Kalium, Rubidium und/oder Cäsium als Promotor in einer Menge von 0,001 bis 0,5 Gew.-% auf einem hitzebeständigen, porösen Trägermaterial, Gewichtsprozente jeweils bezogen auf das Gewicht des Katalysators (Gesamtgewicht des Katalysators), wobei das Silber und der Promotor gleichzeitig auf den Träger aufgebracht worden sind. Das Aufbringen erfolgt durch Imprägnieren des Trägermaterials mit entsprechenden Imprägnierlösungen. Die auf dem Trägermaterial befindliche Silberverbindung wird thermisch in metallisches Silber umgewandelt (reduziert).

Dieser Silberkatalysator besitzt aufgrund der verwendeten Promotoren Kalium, Rubidium und Cäsium eine relativ gute Selektivität. Trotzdem sind weitere Versuche unternommen worden, um mit der Methode des gleichzeitigen Abscheidens dieser Promotoren mit dem Silber auf dem Trägermaterial eine noch weitergehende Steigerung der Selektivität und eine Verbesserung auch der übrigen wichtigen Eigenschaften, wie insbesondere Aktivität und Lebensdauer, zu erreichen. Dazu wird in genannten DE-A-2 734 912 und DE-A-2 951 969 eine spezielle Imprägnierlösung verwendet und nach genannten DE-A-2 951 970 der imprägnierte und aktivierte Katalysator in einer Mühle behandelt, um von seiner Oberfläche 1 bis 10 Gew.-% zu entfernen, womit auch eine hohe Lebensdauer erzielt werden soll.

Schließlich wird in einer Reihe von Druckschriften, beispielsweise in DE-A-2 454 972, DE-A-2 640 540, DE-A- 2 723 918, DE-A-28 19 595 und DE-A-2 820 170 festgestellt, daß man dann zu neuen Silberträgerkatalysatoren mit erhöhter Wirksamkeit kommt, wenn man neben dem Silber mehrere spezielle Promotormetalle auf das Trägermaterial aufbringt, wobei es gleichgültig sein soll, in welcher Reihenfolge Silber und Promotor auf das Trägermaterial abgeschieden werden.

Der Stand der Technik über die Verfahrensweise beim Aufbringen von Silber und Promotor auf das Trägermaterial und bei der Reduktion der aufgebrachten Silberverbindung zu metallischem Silber zeigt, daß bereits mehrere Methoden bekannt sind, die zu wirksamen Silberträgerkatalysatoren führen sollen. Es besteht aber dennoch ein Bedarf nach neuen Silberkatalysatoren mit guten Eigenschaften, insbesondere nach Silberkatalysatoren, die eine hohe Aktivität, Selektivität und Lebensdauer besitzen, zumal die bekannten Herstellungsverfahren teilweise spezielle Imprägnierungen, spezielle Reduktionen und/oder nachträgliche spezielle Behandlungen des fertigen Katalysators erfordern.

Es wurde nun überraschenderweise gefunden, daß Silberträgerkatalysatoren mit hoher Aktivität, Selektivität und Lebensdauer erhalten werden, wenn das gleichzeitige Aufbringen von Silber und Promotor in den erforderlichen Mengen auf das Trägermaterial nicht auf einmal, sondern in zwei Stufen erfolgt und in jeder Stufe eine ganz bestimmte Teilmenge an Silber und Promotor aufgebracht wird. Es war nicht zu erwarten, daß gerade mit dieser Variante des gleichzeitigen Aufbringens von Silber und Promotor eine derart hohe Wirkung erreicht wird, zumal in DE-A-2 951 970 einer wiederholten Imprägnierung des Trägers unter Verwendung von entsprechenden Imprägnierlösungen zum gleichzeitigen Aufbringen der erforderlichen Silber- und Promotormenge keinerlei Bedeutung beigemessen wird und eine Wirksamkeitssteigerung des Katalysators angeblich nur mit der dort beschriebenen Nachbehandlung des Katalysators zur Entfernung seiner Oberflächenhaut erreicht werden kann.

Es wurde ferner gefunden, daß Silberkatalysatoren mit einer hohen Aktivität, Selektivität und Lebensdauer überraschenderweise auch dann erhalten werden, wenn das Aufbringen von Silber und Kalium, Rubidium und/oder Cäsium als Promotor auf das Trägermaterial in der Weise erfolgt, daß zunächst die gesamte Silbermenge und nur ein Teil der gesamten Promotormenge und anschließend die restliche Menge (auf die Gesamtmenge) an Promotor aufgebracht wird.

Es hat sich herausgestellt, daß bei den beiden Verfahren des Aufbringens von Silber und Promotor im Falle des Silbers eine im wesentlichen gleichmäßige Verteilung auf der inneren und äußeren Oberfläche des Trägermaterials vorliegt und im Falle des Promotors die äußere Oberfläche des Trägermaterials eine höhere Konzentration als die innere Oberfläche aufweist.

Der erfindungsgemäße Silberkatalysator, bestehend aus Silber in einer Menge von 3 bis 20 Gew.-% und Kalium, Rubidium und/oder Cäsium als Promotor in einer Menge von 0,003 bis 0,05 Gew.-% auf einem hitzebeständigen, porösen Trägermaterial, Gewichtsprozente jeweils bezogen auf das Gewicht des Katalysators (Gewicht des fertigen Katalysators oder Gesamtgewicht des Katalysators), wobei das Silber und der Promotor auf das Trägermaterial unter Verwendung von Imprägnierlösungen aufgebracht worden sind und die aufgebrachte Silberverbindung zu metallischem Silber reduziert worden ist, ist dadurch gekennzeichnet, daß das Aufbringen von Silber und Promotor und die Reduktion nach einer der beiden nachstehenden Methoden durchgeführt worden sind:

a) gleichzeitiges Aufbringen von 55 bis 85 Gew.-% von der gesamten Silbermenge und 15 bis 45 Gew.-% von der gesamten Promotormenge in einer ersten Imprägnierung,

b) Trocknen des in Schritt a) erhaltenen Produktes,
c) gleichzeitiges Aufbringen des Restes von der gesamten Silbermenge und Promotormenge auf das in Schritt b) erhaltene Produkt in einer zweiten Imprägnierung und
d) Erhitzen des in Schritt c) erhaltenen Produktes zur Reduzierung der aufgebrachten Silberverbindung zu metallischem Silber;

oder

a') gleichzeitiges oder nacheinanderfolgendes Aufbringen der gesamten Silbermenge und 15 bis 45 Gew.-% von der gesamten Promotormenge in einer beziehungsweise in zwei Imprägnierungen,
b') Erhitzen des in Schritt a') erhaltenen Produktes zur Reduzierung der aufgebrachten Silberverbindung zu metallischem Silber,
c') Aufbringen des Restes von der gesamten Promotormenge auf das in Schritt b') erhaltene Produkt in einer weiteren Imprägnierung und
d') Trocknen des in Schritt c') erhaltenen Produktes, und daß im Falle des Silbers eine im wesentlichen gleichmäßige Verteilung auf der inneren und äußeren Oberfläche des Trägermaterials vorliegt und im Falle des Promotors die äußere Oberfläche des Trägermaterials eine höhere Konzentration als die innere Oberfläche aufweist.

Das erfindungsgemäße Verfahren zur Herstellung eines Silberkatalysators, wobei das Silber und der Promotor auf das Trägermaterial unter Verwendung von Imprägnierlösungen aufgebracht werden und die aufgebrachte Silberverbindung zu metallischem Silber reduziert wird, ist dadurch gekennzeichnet, daß es nach einer der beiden nachstehenden Methoden durchgeführt wird:

a) gleichzeitiges Aufbringen von 55 bis 85 Gew.-% von der gesamten Silbermenge und 15 bis 45 Gew.-% von der gesamten Promotormenge in einer ersten Imprägnierung,
b) Trocknen des in Schritt a) erhaltenen Produktes,
c) gleichzeitiges Aufbringen des Restes von der gesamten Silbermenge und Promotormenge auf das in Schritt b) erhaltene Produkt in einer zweiten Imprägnierung und
d) Erhitzen des in Schritt c) erhaltenen Produktes zur Reduzierung der aufgebrachten Silberverbindung zu metallischem Silber;

oder

a') gleichzeitiges oder nacheinanderfolgendes Aufbringen der gesamten Silbermenge und 15 bis 45 Gew.-% von der gesamten Promotormenge in einer beziehungsweise in zwei Imprägnierungen,
b') Erhitzen des im Schritt a') erhaltenen Produktes zur Reduzierung der aufgebrachten Silberverbindung zu metallischem Silber,
c') Aufbringen des Restes von der gesamten Promotormenge auf das im Schritt b') erhaltene Produkt in einer weiteren Imprägnierung und
d') Trocknen des im Schritt c') erhaltenen Produktes.

Die Silbermenge beträgt vorzugsweise 7 bis 14 Gew.-% und die Promotormenge 0,008 bis 0,035 Gew.-%, jeweils bezogen auf das Gewicht des Katalysators.
Der Promotor ist vorzugsweise Cäsium.
Bei der ersten erfindungsgemäßen Methode werden im Schritt a) vorzugsweise 60 bis 75 Gew.-% von der gesamten Silbermenge und 25 bis 40 Gew.-% von der gesamten Promotormenge und im Schritt c) der Rest von der gesamten Silber- und Promotormenge aufgebracht. Bei der zweiten erfindungsgemäßen Methode werden im Schritt a') vorzugsweise 20 bis 35 Gew.-% von der gesamten Promotormenge aufgebracht.
Als Trägermaterial der erfindungsgemäßen Silberkatalysatoren kommen die üblichen, im Handel erhältlichen hitzebeständigen und porösen Materialien in Betracht. Diese sind auch in Gegenwart der bei der Oxidation von Ethylen herrschenden Reaktionsbedingungen und anwesenden chemischen Verbindungen inert. Das Trägermaterial zum Herstellen der erfindungsgemäßen Silberkatalysatoren ist nicht kritisch, geeignete Träger sind beispielsweise Kohle, Korund, Siliciumcarbid, Siliciumdioxid, Aluminiumoxid und Gemische aus Aluminiumoxid und Siliciumdioxid. Bevorzugt ist α-Aluminiumoxid, da es einen weitgehend gleichmäßigen Porendurchmesser aufweist. Es besitzt eine spezifische Oberfläche von 0,1 bis 1 $m^2/g$, vorzugsweise 0,2 bis 0,6 $m^2/g$ (gemessen nach der bekannten B.E.T.-Methode), ein spezifisches Porenvolumen von 0,1 bis 1 $cm^3/g$, vorzugsweise 0,2 bis 0,6 $cm^3/g$ (gemessen nach der bekannten Quecksilber- oder Wasseradsorptionsmethode), eine scheinbare Porosität von 20 bis 70 Vol.-%, vorzugsweise 40 bis 60 Vol.-% (gemessen nach der bekannten Quecksilber- oder Wasseradsorptionsmethode), einen mittleren Porendurchmesser von 0,3 bis 15 µm, vorzugsweise 1 bis 10 µm, und einen prozentuellen Anteil an Poren mit einem Durchmesser von 0,03 bis 10 µm von mindestens 50 Gew.-% (Porendurchmesser und Porendurchmesserverteilung werden bekanntlich aus der spezifischen Oberfläche und der scheinbaren Porosität ermittelt).
Das Trägermaterial wird vorteilhafterweise in Form von Granulaten, Kugeln, Ringen, Pellets und dergleichen

eingesetzt. Beispiele von bevorzugten α-Aluminiumoxid- oder α-Aluminiumoxid enthaltenden Trägermaterialien sind die von der Firma Norton Company angebotenen Typen mit der Bezeichnung SA 5551 und SA 5552 oder die SAHM-Typen der Firma United Catalyst.

Das erfindungsgemäße Aufbringen von Silber und Promotor auf das Trägermaterial erfolgt mit Hilfe des bekannten Imprägnierverfahrens. Danach wird das Trägermaterial mit Lösungen in Kontakt gebracht, vorzugsweise durch Eintauchen getränkt, die aus einem Lösungsmittel und aus einer für die Silberabscheidung und für die Promotorabscheidung auf das Trägermaterial ausreichenden Menge an Silberverbindung und/oder Promotorverbindung bestehen, worauf das Trägermaterial von der überschüssigen Lösung abgetrennt und getrocknet wird.

Nach der ersten erfindungsgemäßen Methode wird das Trägermaterial zunächst so imprägniert, daß 55 bis 85 Gew.-%, vorzugsweise 60 bis 75 Gew.-%, von der gesamten Silbermenge und 15 bis 45 Gew.-%, vorzugsweise 25 bis 40 Gew.-%, von der gesamten Promotormenge aufgebracht werden. Nach dieser ersten Imprägnierung wird auf das getrocknete Trägermaterial durch eine zweite Imprägnierung der Rest (auf die jeweilige Gesamtmenge) an Silber und Promotor aufgebracht. Die Lösungen für die beiden erfindungsgemäßen Imprägnierschritte bestehen demnach im wesentlichen aus einem Lösungsmittel und einer ausreichenden Menge an Silberverbindung und Promotorverbindung.

Nach der zweiten erfindungsgemäßen Methode wird das Trägermaterial im Schritt a') so imprägniert, daß die gesamte Silbermenge, aber nur 15 bis 45 Gew.-%, vorzugsweise 20 bis 35 Gew.-%, von der gesamten Promotormenge aufgebracht werden. Dies kann dadurch erreicht werden, daß man das Trägermaterial zuerst mit einer Lösung mit Lösung mit mindestens einer Promotorverbindung imprägniert (Variante 1) oder mit einer Lösung, die mindestens eine Silberverbindung und mindestens eine Promotorverbindung zugleich enthält (gleichzeitige Abscheidung von Silber- und Promotorverbindung, Variante 2). Die Lösungen für den Imprägnierschritt a') bestehen demnach im wesentlichen aus einem Lösungsmittel und einer jeweils ausreichenden Menge an Silberverbindung und Promotorverbindung. Von den beiden Varianten des Schrittes a') ist die gleichzeitige Abscheidung von Silber- und Promotorverbindung bevorzugt. Je nach eingesetztem Trägermaterial können die zweckmäßig anzuwendenden Konzentrationen an Silberverbindung und Promotorverbindung in den Lösungen durch Vorversuche und analytische Bestimmung der Mengen der tatsächlich niedergeschlagenen Verbindungen leicht und schnell ermittelt werden.

Die Imprägnierungen können nach einer der üblichen Arbeitsweisen durchgeführt werden. Sie erfolgen zweckmäßigerweise dadurch, daß man das Trägermaterial mit der Imprägnierlösung in einem Gefäß tränkt (eintaucht, übergießt), wobei die Lösung durch Absorption und/oder durch Kapillarwirkung in die Poren des Trägermaterials eindringt und sich Silberverbindung und Promotorverbindung abscheiden. Die überschüssige Imprägnierlösung wird dann abgetrennt (z. B. durch Abgießen, Abtropfen, Abfiltrieren oder Abzentrifugieren), worauf das vollgesogene Trägermaterial getrocknet wird. Die Menge an Imprägnierlösung wird im allgemeinen so gewählt, daß ein volumenmäßiger Überschuß, bezogen auf das zu imprägnierende Trägermaterialvolumen, vorliegt. Im allgemeinen wird das 0,5- bis 3-fache, vorzugsweise das 1- bis 2-fache Volumen an Imprägnierflüssigkeit, bezogen auf das Volumen an Trägermaterial, genommen. Die Imprägnierzeit, das ist die Zeit, in der das Trägermaterial mit der Imprägnierflüssigkeit in Kontakt bleibt, ist klarerweise so zu wählen, daß die erforderliche Menge an abzuscheidender Silberverbindung und Promotorverbindung auf den Träger aufgebracht wird. Sie beträgt im allgemeinen 5 bis 60 Minuten und hängt insbesondere von der Konzentration der Imprägnierlösung an Silber- und Promotorverbindung, von dem verwendeten Trägermaterial und von dessen jeweiligem Saugvermögen ab. Die beim Imprägnieren angewandte Temperatur kann innerhalb weiter Grenzen variieren. Im allgemeinen wird bei Raumtemperatur imprägniert. Um den Imprägniervorgang zu beschleunigen, können auch höhere Temperaturen angewandt werden. Die Imprägniertemperatur beträgt demnach in der Regel 15 bis 80 °C, vorzugsweise 20 bis 50 °C. Die Imprägnierung wird in der Regel bei Atmosphärendruck durchgeführt.

Die Trocknung der von den Imprägnierungen jeweils erhaltenen Produkte (imprägnierten Trägermaterialien) erfolgt im allgemeinen bei einer Temperatur von 20 bis 150 °C, vorzugsweise 50 bis 120 °C. Die Abdampfung des Lösungsmittels kann beispielsweise mit Hilfe von Hordentrocknern, Drehrohröfen oder durch Überleiten von heißen Inertgasen, wie Stickstoff und/oder Kohlendioxid, durchgeführt werden. Die Temperatur richtet sich klarerweise nach dem Siedepunkt des Lösungsmittels der Imprägnierflüssigkeit.

Die Reduzierung der auf dem Trägermaterial abgeschiedenen Silberverbindung zu metallischem Silber erfolgt durch Erhitzen. Zur thermischen Reduzierung der Silberverbindungen ist im allgmeinen eine Temperatur von 170 bis 400°C, vorzugsweise 200 bis 350°C, erforderlich. Das Erhitzen auf diese Temperaturen kann beispielsweise in einem Hordentrockner, Drehrohrofen, elektrisch beheizten Rohr oder durch Überleiten eines entsprechend erhitzten Inertgases, wie Luft, Stickstoff, Kohlendioxid oder einer Mischung davon, durchgeführt werden. Die Umwandlung der Silberverbindung in metallisches Silber kann auch mit Hilfe von überhitztem Wasserdampf vorgenommen werden. Die bei den genannten Temperaturen erforderliche Erhitzungszeit beträgt im allgemeinen 0,2 bis 5 Stunden, vorzugsweise soll diese Zeit 0,3 bis 1 Stunde betragen. Durch die Zersetzung der Silberverbindung bildet sich ein festhaftender Niederschlag von metallischen Silberteilchen auf dem Trägermaterial (die Promotorverbindungen werden nicht zum entsprechenden Metall reduziert, die Alkalimetalle Kalium, Rubidium und Cäsium liegen also im wesentlichen in Form ihrer Kationen und nicht als freies Alkalimetall vor). Das Silber (die Silberteilchen) liegt in der Regel in Form von festhaftenden, im wesentlichen gleichmäßig verteilten, nicht zusammenhängenden, diskreten Teilchen mit einem Durchmesser von weniger als 1,5 µm vor. Im allgemeinen weisen die Silberteilchen Durchmesser von 0,1 bis 1 µm und einen

mittleren Durchmesser von 0,2 bis 0,7 µm auf.

Nachstehend werden geeignete Silberverbindungen, Silberkomplexbildner, Promotorverbindungen und geeignete Lösungsmittel sowie zweckmäßige Imprägnierlösungen angegeben.

Als Silberverbindungen werden vorzugsweise Silbersalze eingesetzt. Geeignete anorganische Salze sind beispielsweise Silbernitrat und Silbercarbonat. Geeignete organische Salze sind beispielsweise solche von ein- oder mehrbasischen Carbonsäuren und Hydroxycarbonsäuren mit bis zu 6 Kohlenstoffatomen, wie Silberacetat, Silberlactat und Silberoxalat. Die Silberverbindungen sind wasserlöslich und in der Hitze zu metallischem Silber zersetzbar.

Es ist vorteilhaft, einen Silber-Komplexbildner einzusetzen, um die Löslichkeit der Silberverbindung zu erhöhen. Solche Komplexbildner sind bekanntlich Ammoniak, Aminosäuren und/oder Amine, wie Alkylendiamine mit 2 bis 4 Kohlenstoffatomen, beispielsweise Ethylendiamin, Alkanolamine mit 2 bis 4 Kohlenstoffatomen, beispielsweise Ethanolamin, Mono-, Di- und Trialkylamine mit 1 bis 4 Kohlenstoffatomen (in der Alkylgruppe), beispielsweise Methylamin, Isopropylamin, Isobutylamin und sekundäres Butylamin, und Polyamine. Unter den genannten Komplexbildnern sind die Alkylamine bevorzugt, vorzugsweise die Monoalkylamine mit 1 bis 4 Kohlenstoffatomen. Die Komplexbildner werden in einer solchen Menge zugefügt, die ausreicht, um die Silberverbindung quantitativ in den Silberamino-Komplex überzuführen. Dazu ist in der Regel ein geringer molarer Überschuß an Aminogruppen notwendig. Da ein Silberkation 2 Aminogruppen bindet, ist der Einsatz von mindestens 2 Aminogruppen pro mol Silberkation erforderlich.

Als Promotorverbindungen sind Salze, Hydroxide und Oxide der genannten Metalle Kalium, Rubidium und Cäsium geeignet. Die Salze sind bevorzugt, wobei es nicht kritisch ist, welches Anion im Salz vorliegt. Als anorganische Salze seien das Nitrat, Chlorid, Carbonat und das Phosphat genannt. Als organische Salze seinen jene von ein- und mehrbasischen Carbonsäuren und Hydroxycarbonsäuren mit bis zu 6 Kohlenstoffatomen, beispielsweise das Formiat, Acetat, Oxalat, Citrat und Lactat, genannt. Die Promotorverbindungen sind ebenso wie die Silberverbindungen wasserlöslich.

Geeignete Lösungsmittel für die Silber- und Promotorverbindungen sind Wasser, aliphatische Alkohole mit 1 bis 5, vorzugsweise 1 bis 3 Kohlenstoffatomen, wie Methanol, Ethanol, Propanol und Isopropanol, aliphatische Ketone mit 3 bis 5 Kohlenstoffatomen, wie Aceton, aliphatische und cyclische Ether, wie Diethylether, Methylethylether, Dipropylether und Dioxan, Ester, wie Methylacetat und Ethylacetat, Säureamide, wie Dimethylformamid, und Nitrile, wie Acetonitril, sowie Mischungen davon.

Im Falle der Anwesenheit von Silberverbindungen und Silber- und Promotorverbindungen ist Wasser und im Falle der Anwesenheit von Promotorverbindungen allein sind die aliphatischen Alkohole mit 1 bis 3 Kohlenstoffatomen (mit gegebenenfalls einer geringen Menge Wasser als Lösungsvermittler) als Lösungsmittel bevorzugt.

Für den Schritt a) der erfindungsgemäßen Katalysatorherstellung besteht eine zweckmäßige Imprägnierlösung im wesentlichen aus (1) einem (wasserlöslichen und hitzezersetzbaren) Silbersalz in einer Menge von 30 bis 40 Gew.-%, (2) mindestens einem (wasserlöslichen) Promotorsalz, vorzugsweise einem Cäsiumsalz, in einer Menge von 0,02 bis 0,04 Gew.-%, (3) Wasser in einer Menge von 20 bis 30 Gew.-%, und (4) einem Monoalkylamin mit 1 bis 4 Kohlenstoffatomen als Komplexbildner für das Silbersalz in einer Menge von 30 bis 40 Gew.-%, Gewichtsprozente bezogen auf das Gewicht der Lösung. Für den Schritt c) besteht sie im wesentlichen aus (1) einem (wasserlöslichen und hitzezersetzbaren) Silbersalz in einer Menge von 30 bis 40 Gew.-%, (2) mindestens einem (wasserlöslichen) Promotorsalz, vorzugsweise einem Cäsiumsalz, in einer Menge von 0,045 bis 0,07 Gew.-%, (3) Wasser in einer Menge 20 bis 30 Gew.-% und (4) einem Monoalkylamin mit 1 bis 4 Kohlenstoffatomen als Komplexbildner für das Silbersalz in einer Menge von 30 bis 40 Gew.-%, Gewichtsprozente bezogen auf das Gewicht der Lösung. (Es versteht sich von selbst, daß die konkrete Konzentration an Silbersalz und Promotorsalz in den beiden Imprägnierlösungen sich nach derjenigen Menge an Silber und Promotor richtet, die man auf den Träger aufbringen will.) Es ist nicht notwendig, das im Schritt c) erhaltene Produkt, bevor es gemäß Schritt d) behandelt wird, eigens zu trocknen. Die Abdampfung des Lösungsmittels erfolgt ohnehin beim Aufheizen auf Reduktionstemperatur im Schritt d).

Für den Schritt a') der erfindungsgemäßen Katalysatorherstellung werden zweckmäßigerweise die nachstehenden Imprägnierlösungen verwendet: Die Lösung zum Aufbringen des Silbers auf das Trägermaterial gemäß Variante 1 besteht im wesentlichen aus (1) einem (wasserlöslichen und hitzezersetzbaren) Silbersalz in einer Menge von 30 bis 40 Gew.-%, (2) Wasser in einer Menge von 20 bis 30 Gew.-%, und (3) einem Monoalkylamin mit 1 bis 4 Kohlenstoffatomen als Komplexbildner für das Silbersalz in einer Menge von 30 bis 40 Gew.-%; die Lösung zum Aufbringen des Promotors gemäß Variante 1 besteht im wesentlichen aus (1) mindestens einem (wasserlöslichen und/oder alkohollöslichen) Promotorsalz, vorzugsweise einem Cäsiumsalz in einer Menge von 0,01 bis 0,03 Gew.-%, (2) Wasser in einer Menge von 0 bis 5 Gew.-%, und (3) einem aliphatischen Alkohol mit 1 bis 3 Kohlenstoffatomen als Restmenge, das ist Prozentmenge auf 100 Gew.-%; die Lösung zum gleichzeitigen Aufbringen von Silber- und Promotorverbindung gemäß Variante 2 des Schrittes a') besteht im wesentlichen aus (1) einem (wasserlöslichen und hitzezersetzbaren) Silbersalz in einer Menge von 30 bis 40 Gew.-%, (2) mindestens einem (wasserlöslichen) Promotorsalz, vorzugsweise einem Cäsiumsalz, in einer Menge von 0,01 bis 0,03 Gew.-%, (3) Wasser in einer Menge von 20 bis 30 Gew.-%, und (4) einem Monoalkylamin mit 1 bis 4 Kohlenstoffatomen als Komplexbildner für das Silbersalz in einer Menge von 30 bis 40 Gew.-%, Gewichtsprozente bezogen auf das Gewicht der Lösung. Eine zweckmäßige Imprägnierlösung für den Schritt c') besteht im wesentlichen aus (1) mindestens einem (wasserlöslichen und/oder alkohollöslichen) Promotorsalz, vorzugsweise einem Cäsiumsalz, in einer Menge von 0,04 bis 0,08 Gew.-%, (2) Wasser in einer

Menge von 0 bis 5 Gew.-%, und (3) einem aliphatischen Alkohol mit 1 bis 3 Kohlenstoffatomen als Restmenge, das ist Prozentmenge auf 100 Gew.-%, Gewichtsprozente bezogen auf das Gewicht der Lösung. (Es versteht sich von selbst, daß die konkrete Konzentration an Silbersalz und Promotorsalz in den Imprägnierlösungen sich nach derjenigen Menge an Silber und Promotor richtet, die man auf den Träger aufbringen will.)

Bei der Variante 1 des Schrittes a') wird zweckmäßigerweise eine Zwischentrocknung durchgeführt, das heißt, daß nach der Imprägnierung mit der Lösung der Silberverbindung und vor der Imprägnierung mit der Promotorverbindung getrocknet wird. Es ist nicht notwendig, das im Schritt a') erhaltene Produkt, bevor es gemäß Schritt b') behandelt wird, einer eigenen Trocknung zu unterwerfen. Die Abdampfung des Lösungsmittels vom Trägermaterial kann nämlich auch im Schritt b') erfolgen, in dem ohnehin auf höhere Temperaturen erhitzt wird.

Bei der zweiten Methode des erfindungsgemäßen Verfahrens hat es sich in manchen Fällen als vorteilhaft erwiesen, den nach den Schritten a') bis d') erhaltenen Silberkatalysator einer Wäschebehandlung zu unterwerfen, in der der Katalysator mit einem Lösungsmittel kontaktiert wird, in dem die auf den Träger aufgebrachte Promotorverbindung löslich ist. Die Wäschebehandlung wird vor allem dann durchgeführt, wenn der fertige Katalysator eine zu hohe Menge an Promotorverbindung enthält. Durch das Waschen läßt sich nämlich die überschüssige Menge in einfacher Weise entfernen. Die Wäschebehandlung kann auch deswegen durchgeführt werden, um eine bestimmte optimale Alkalimetallkonzentration im fertigen Katalysator schnell und einfach herzustellen.

Als Waschflüssigkeit werden vorzugsweise Wasser, aliphatische Alkohole mit 1 bis 4 Kohlenstoffatomen oder Mischungen davon eingesetzt. Eine besonders bevorzugte Waschflüssigkeit besteht aus Methanol, Ethanol, Propanol und/oder Isopropanol und Wasser in einer Menge von 0 bis 20 Gew.-%, bezogen auf das Gewicht der Waschflüssigkeit.

Das Waschen selbst kann mit den an sich bekannten Arbeitsweisen durchgeführt werden, wobei der Katalysator mit der Waschflüssigkeit in Berührung gebracht und anschließend davon abgetrennt und getrocknet wird. Nach einer bevorzugten Arbeitsweise erfolgt das Waschen dadurch, daß der Katalysator in einem Gefäß mit der Waschflüssigkeit überschüttet und anschließend davon durch Zentrifugieren, Filtrieren, Abnutschen oder einfach Abgießen (Abdekantieren) abgetrennt wird. Die Zeit, die der Katalysator mit der Waschflüssigkeit in Kontakt gelassen wird, ist an sich nicht kritisch. Sie hängt vor allem von der abzulösenden Menge an Promotorverbindung ab und liegt im allgemeinen bei 0,2 bis 20 Minuten, vorzugsweise bei 0,5 bis 5 Minuten. In der Regel genügt es, den Waschvorgang einmal vorzunehmen. Es kann jedoch von Vorteil sein, ein absatzweises Waschen durchzuführen, indem der Katalysator 2- bis 5-mal, zweckmäßigerweise 2- bis 3-mal, gewaschen wird, wobei vorzugsweise jeweils eine frische (neue, noch nicht gebrauchte) Waschflüssigkeit verwendet wird. Die Menge an Waschflüssigkeit (für ein- oder mehrmalige Wäsche) richtet sich nach der zu waschenden Katalysatormenge und ist natürlich so zu bemessen, daß der Katalysator mit der Flüssigkeit, gegebenenfalls unter Rühren in Berührung kommt. Sie beträgt (in Volumenteilen) zweckmäßigerweise mindestens etwa 1/3 der Menge an Katalysator (in Volumenteilen), vorzugsweise wird etwa die 1- bis 3-fache Menge an Waschflüssigkeit eingesetzt. Die Temperatur und der Druck während des Waschens ist nicht kritisch; es kann drucklos oder unter Druck gewaschen werden. Im allgemeinen wird das Waschen bei einer Temperatur von 15 bis 80° C, vorzugsweise 20 bis 50° C, durchgeführt.

Die Trocknung des gewaschenen Katalysators kann beispielsweise mit Hilfe eines Inertgases wie Stickstoff, Kohlendioxid, Luft oder Gemische davon und/oder durch Erwärmen des Katalysators erreicht werden, wobei zur Beschleunigung des Trocknungsvorganges auch Unterdruck angewendet werden kann. Die Temperatur, bei der die Trocknung durchgeführt wird, ist nicht kritisch. Sie richtet sich zweckmäßigerweise nach dem Siedepunkt der eingesetzten Waschflüssigkeit. Zweckmäßige Trocknungstemperaturen sind 20 bis 150° C, vorzugsweise 50 bis 120° C.

Beim erfindungsgemäßen Silberkatalysator ist das Silber auf den inneren und äußeren Oberflächen des Trägermaterials im wesentlichen gleichmäßig verteilt. Das Alkalimetall (die Alkalimetallverbindung) hingegen ist ungleichmäßig (unsymmetrisch) verteilt. In den äußeren Schichten des Trägerkörpers ist eine höhere Promotorkonzentration vorhanden als in den inneren Schichten. Der Promotor liegt also in Form eines solchen Konzentrationsgefälles vor, daß in jedem Trägerkörper außen eine höhere Promotorkonzentration vorhanden ist als innen.

Der erfindungsgemäße Silberkatalysator besitzt eine hohe aktivität und Selektivität und eine lange Lebensdauer. Aufgrund seiner langen Lebensdauer behält er die hohe Wirksamkeit über eine relativ lange Gebrauchszeit bei. Das bringt den weiteren Vorteil mit sich, daß beim erfindungsgemäßen Silberkatalysator ein Austausch oder eine Regenerierung erst nach relativ großen Zeitabständen erforderlich ist.

Das erfindungsgemäße Verfahren zur Herstellung von Silberkatalysatoren ist einfach und leicht durchzuführen. Es enthält keine komplizierten oder aufwendigen Verfahrensschritte, und es sind auch keine speziellen Imprägnierlösungen erforderlich.

Die bei der erfindungsgemäßen Verwendung des neuen Silberkatalysators anzuwendenden Bedingungen, wie Temperatur, Druck, Verweilzeit, Verdünnungsmittel, Bremssubstanzen zur Kontrolle der katalytischen Oxidation von Ethylen mit Sauerstoff, Recycling, verfahrenstechnische Maßnahmen zur Erhöhung der Ethylenoxidausbeute und dergleichen, sind an sich bekannt. Die Reaktionstemperatur liegt im allgemeinen bei 150 bis 400° C, vorzugsweise 200 bis 300° C, der Reaktionsdruck bei 0,15 bis 3 MPa, vorzugsweise 1 bis 2 MPa. Die eingesetzte Beschickungsmischung enthält im allgemeinen 5 bis 30 Mol-% Ethylen, 3 bis 15 Mol-% Sauerstoff und als Rest inerte Gase, wie Stickstoff, Kohlendioxid, Wasserdampf, Methan, Ethan, Argon und

dergleichen, sowie Vinylchlorid, 1,2 Dichlorethan und dergleichen als Bremssubstanzen. Aus dem Umsetzungsprodukt wird das Ethylenoxid in üblicher Weise isoliert und das Gasgemisch wie üblich eventuell gereinigt und wieder zurückgeführt.

Eine bevorzugte Ausführungsform der Verwendung der erfindungsgemäßen Silberkatalysatoren ist seine Verwendung zur Herstellung von Ethylenoxid durch direkte Oxidation von Ethylen mit molekularem Sauerstoff bei einer Temperatur von 200 bis 270° C unter Anwendung eines Katalysatorfestbettes.

Die Erindung wird nun an Beispielen noch näher erläutert.

Die Beispiele 1 bis 4 beziehen sich auf das erste Verfahren und die Beispiele 5 bis 8 auf das zweite Verfahren der erfindungsgemäßen Katalysatorherstellung.

**Beispiel 1**

Zur Herstellung eines erfindungsgemäßen Katalysators wurde eine Lösung aus

13,03 g (37,9441 Gew.-%) Silbernitrat
9,10 g (26,4997 Gew.-%) destilliertem Wasser
0,01 g ( 0,0291 Gew.-%) Cäsiumnitrat
12,20 g (35,5271 Gew.-%) Isobutylamin

bereitet.

In diese Lösung wurde das Trägermaterial SA 5552 der Firma Norton Company, nämlich α-Aluminiumoxid in Form von Zylindern mit einer spezifischen Oberfläche von 0,3 $m^2/g$, 15 Minuten lang bei Raumtemperatur vollständig eingetaucht. Nach dem Abtropfen der überschüssigen Imprägnierlösung über einem Sieb wurde das feuchte Trägermaterial 30 Minuten lang bei 105° C in einer Luft-Stickstoff-Atmosphäre getrocknet (Verfahrensschritte a) und b).

Nach dem Abkühlen des halbfertigen Katalysators wurde dieser mit einer Lösung, bestehend aus

13,03 g (36,6557 Gew.-%) Silbernitrat
10,00 g (28,1318 Gew.-%) destilliertem Wasser
0,017 g ( 0,0478 Gew.-%) Cäsiumnitrat
12,50 g (35,1647 Gew.-%) Isobutylamin

nochmals, wie oben beschrieben, imprägniert und getrocknet (Verfahrensschritt c).

Der Trocknung schloß sich eine halbstündige Reduktion in einem auf 300° C vorgeheizten Drehrohrofen an; der Often wurde mit einem Luft-Stickstoff-Gemisch durchstömt (Verfahrensschritt d). Erhalten wurde ein Silberkatalysator mit 11,4 Gew.-% Silber und 0,015 Gew.-% Cäsium.

Durch die erste Imprägnierung wurden 74 Gew.-% der gesamten Silbermenge und 33 Gew.-% der gesamten Cäsiummenge und mit der zweiten Imprägnierung die jeweilige Restmenge aufgebracht. 20 ml des so hergestellten Katalysators wurden in einen Edelstahl-Druckreaktor eingefüllt und bei 210° C und 1,3 MPa Gasdruck mit einem Betriebsgas, bestehend aus 30 Vol.-% Ethylen, 50 Vol.-% Methan, 8,5 Vol.-% Sauerstoff, 0,0003 Vol.-% Vinylchlorid und dem Rest Stickstoff beschickt. Die Raum-Zeit-Geschwindigkeit betrug dabei 3000 Normalliter Gas pro Liter Katalysator pro Stunde.

Das den Reaktor verlassende Gas enthielt 1,5 Vol.-% Ethylenoxid. Daraus wurde die Selektivität (= mole gebildetes Ethylenoxid pro mol umgesetztes Ethylen) von 82,1 % bei 6 % Ethylenumsatz errechnet.

In einem Dauerversuch bei den obengenannten Bedingungen wurde im Laufe von 4 Monaten ein Abfall der Selektivität um nur 0,3 Punkte (das ist auf 81,8 %) festgestellt.

**Beispiel 2 (Vergleichsbeispiel)**

Bei diesem Vergleichsbeispiel wurde in nur einer Imprägnierung die gesamte Cäsiummenge und Silbermenge aufgetragen.

Das Trägermaterial von Beispiel 1 wurde nur einmal mit einer Lösung, bestehend aus

31,5 g (49,4910 Gew.-%) Silbernitrat
15,6 g (24,5098 Gew.-%) destilliertem Wasser
0,048 g ( 0,0754 Gew.-%) Cäsiumnitrat
16,5 g (25,9238 Gew.-%) Ethylendiamin

getränkt und wie in Beispiel 1 getrocknet und reduziert. Der fertige Katalysator enthielt 11,3 Gew.-% Silber und 0,016 Gew.-% Cäsium.

Bei einem Langzeittest mit diesem Katalysator über 4 Monate mit den Bedingungen des Beispiels 1 wurde

folgendes festgestellt:
erforderliche Temperatur für 1,5 Vol.-%

| | |
|---|---|
| Ethylenoxid | 218°C |
| Selektivität bei 6 % $C_2H_4$-Umsatz | 79,3 % |
| Selektivität nach 1 Monat | 79,1 % |

**Beispiel 3**

Bei diesem erfindungsgemäßen Beispiel wurde eine Lösung aus

12,000 g (37,4859 Gew.-%) Silbernitrat
9,000 g (28,1145 Gew.-%) destilliertem Wasser
0,012 g ( 0,0375 Gew.-%) Cäsiumacetat
11,000 g (34,3621 Gew.-%) sekundärem Butylamin

bereitet.

In diese Lösung wurde das Trägermaterial SAHM der Firma United Catalyst, nämlich α-Aluminiumoxid in Form von Kugeln mit 8 mm Durchmesser und einer spezifischen Oberfläche von 0,2 $m^2/g$, 10 Minuten lang, wie in Beispiel 1 beschrieben, eingetaucht und getrocknet.

Nach dem Auskühlen des halbfertigen Katalysators wurde dieser mit einer Lösung, bestehend aus

12,000 g (37,4789 Gew.-%) Silbernitrat
9,000 g (28,1092 Gew.-%) destilliertem Wasser
0,018 g ( 0,0562 Gew.-%) Cäsiumacetat
11,000 g (34,3557 Gew.-%) sekundärem Butylamin

nochmals wie oben beschrieben imprägniert und getrocknet. Der Trocknung schloß sich eine 20 Minuten lange Reduktion in einem auf 280°C vorgeheizten Glasrohr als Ofen an (der Ofen wurde mit 40 Liter Luft und 20 Liter Stickstoff pro Stunde durchströmt).

Erhalten wurde ein Silberkatalysator mit 11,5 Gew.-% Silber und 0,019 Gew.-% Cäsium.

Durch die erste Imprägnierung wurden 68 Gew.-% des gesamten Silbers und 37 Gew.-% des gesamten Cäsiums und mit der zweiten Imprägnierung die jeweilige Restmenge aufgebracht. 20 ml des fertigen Katalysators wurden, wie in Beispiel 1 beschrieben, 4 Monate in der Druckapparatur einem Test unterworfen, wobei folgendes Ergebnis festgestellt wurde:

erforderliche Temperatur für

| | |
|---|---|
| 1,5 Vol.-% Ethylenoxid | 222°C |
| Selektivität bei 6 % $C_2H_4$-Umsatz | 81,8 % |
| Selektivität nach 4 Monaten | 81,5 %. |

**Beispiel 4**

In diesem Beispiel wurde als Promotor ein Cäsium- und Rubidiumsalz eingesetzt. Im übrigen wurde wie in Beispiel 3 vorgegangen.

Die Lösung für die erste Imprägnierung hatte die folgende Zusammensetzung:

| | | |
|---|---|---|
| 12,000 g | (37,4906 Gew.-%) | Silbernitrat |
| 9,000 g | (28,1180 Gew.-%) | destilliertes Wasser |
| 0,005 g | ( 0,0156 Gew.-%) | Cäsiumcarbonat |
| 0,003 g | ( 0,0094 Gew.-%) | Rubidiumnitrat |
| 11,000 g | (34,3664 Gew.-%) | sekundäres Butylamin. |

Lösung für die zweite Imprägnierung:

| | | |
|---|---|---|
| 12,000 g | (36,8992 Gew.-%) | Silbernitrat |
| 9,500 g | (29,2119 Gew.-%) | Wasser |
| 0,012 g | ( 0,0369 Gew.-%) | Cäsiumcarbonat |
| 0,009 g | ( 0,0277 Gew.-%) | Rubidiumnitrat |
| 11,000 g | (33,8243 Gew.-%) | sekundäres Butylamin. |

Der fertige Katalysator enthielt 11,2 Gew.-% Silber, 0,013 Gew.-% Cäsium und 0,008 Gew.-% Rubidium. Dabei wurden im ersten Imprägnierschritt 71 Gew.-% des gesamten Silbers und 25 Gew.-% des gesamten Promotors und die Restmengen mit der zweiten Imprägnierung aufgebracht.

Der Test über 2 Monate, wie in Beispiel 1 beschrieben, zeigte folgendes Ergebnis:
erforderliche Temperatur für

| | |
|---|---|
| 1,5 Vol.-% Ethylenoxid | 226°C |
| Selektivität bei 6 % $C_2H_4$-Umsatz | 81,7 % |
| Selektivität nach 2 Monaten | 81,5 %. |

**Beispiel 5**

Zur Herstellung eines erfindungsgemäßen Katalysators wurde das Trägermaterial SA 5552 der Firma Norton Company, das ist α-Aluminiumoxid in Kugelform mit 8 mm Durchmesser und einer spezifischen Oberfläche von 0,3 $m^2/g$, eingesetzt. Das in einem Glaskolben evakuierte Trägermaterial wurde mit einer Silber- und Cäsiumsalzlösung, bestehend aus

| | | |
|---|---|---|
| 30,000 g | (38,4556 Gew.-%) | Silbernitrat |
| 20,000 g | (25,6371 Gew.-%) | destilliertem Wasser |
| 28,000 g | (35,8919 Gew.-%) | Isobutylamin |
| 0,012 g | ( 0,0154 Gew.-%) | Cäsiumnitrat |

vollständig übergossen. Nach 15 Minuten Standzeit wurde die überschüssige Lösung abdekantiert. Es wurde 45 Minuten lang in einem stickstoff-gespülten Trockenschrank bei 110°C getrocknet (Verfahrensschritt a'). Die trockene Probe wurde anschließend in einem Glasrohr, durch das ein Luft-Stickstoff-Gemisch (40 Liter Luft und 20 Liter Stickstoff pro Stunde) strömte, 30 Minuten lang auf 280°C erhitzt. Nach der hierbei erfolgten Reduktion des Silbersalzes wurde ein Katalysator mit 8,6 Gew.-% Silber und 0,0038 Gew.-% Cäsium erhalten (Verfahrensschritt b').

Der auf Raumtemperatur abgekühlte Rohkatalysator wurde nun (Verfahrensschritt c') mit der folgenden Cäsiumsalzlösung getränkt:

| | |
|---|---|
| Methanol | 95,000 Gew.-% |
| Cäsiumnitrat | 0,045 Gew.-% |
| destilliertes Wasser | 4,955 Gew.-% |

Dazu wurde er in einem Gefäß mit der Lösung vollständig bedeckt und 10 Minuten lang bei Raumtemperatur stehengelassen. Nach dem Abdekantieren und Trocknen bei 110°C im stickstoff-gespülten Trockenschrank (Verfahrensschritt d') wurde ein Katalysator mit 0,0125 Gew.-% Cäsium und 8,6 Gew.-% Silber erhalten (mit der ersten Imprägnierung wurden also die gesamte Silbermenge und 30 Gew.-% von der gesamten Cäsiummenge und mit der zweiten Imprägnierung die Restmenge an Cäsium aufgebracht).

25 ml des so hergestellten Katalysators wurden in einem Reaktor bei 200°C und Normaldruck mit einem Gasgemisch, bestehend aus 30 Vol.-% Ethylen, 50 Vol.-% Methan, 8,5 Vol.-% Sauerstoff, 0,0003 Vol.-% Vinylchlorid, Rest Stickstoff, getestet. Die Raum-Zeit-Geschwindigkeit betrug 400 Normalliter Gas pro Liter Katalysator pro Stunde. Das den Reaktor verlassende Gas enthielt 1,3 Vol.-% Ethylenoxid. Daraus wurde die Selektivität (das ist mole gebildetes Ethylenoxid pro mol umgesetztes Ethylen) von 81,5 % bei 5 % Ethylenumsatz errechnet. In einem Dauerversuch bei den genannten Bedingungen wurde im Laufe von 3

Monaten ein Abfall der Selektivität um nur 0,3 Punkte auf 81,2 % festgestellt.

**Beispiel 6**

Bei diesem erfindungsgemäßen Beispiel wurde das Trägermaterial der SAHM-Type der Firma United Catalyst, ein α-Aluminiumoxid in Form von Kugeln mit 8 mm Durchmesser und mit einer spezifischen Oberfläche von 0,2 $m^2/g$, eingesetzt. Es wurde in einem Becherglas mit einer Lösung aus

30,000 g (39,4659 Gew.-%) Silbernitrat
20,000 g (26,3106 Gew.-%) destilliertem Wasser
22,000 g (28,9417 Gew.-%) sekundärem Butylamin
4,000 g ( 5,2621 Gew.-%) Ethylendiamin
0,015 g ( 0,0197 Gew.-%) Cäsiumnitrat

vollständig übergossen und 15 Minuten lang stehengelassen. Nach dem Abdekantieren der überschüssigen Imprägnierlösung wurde 30 Minuten lang in einem Trockenschrank unter einer Stickstoff-Atmosphäre bei 100° C getrocknet (Verfahrensschritt a').

Zur Reduzierung des auf dem Träger befindlichen Silbernitrates zu metallischem Silber wurde in einem Trockenschrank (der mit 80 Liter Luft und 60 Liter Stickstoff pro Stunde gespült wurde) 1 Stunde lang auf 250° C erhitzt (Verfahrensschritt b').

Der erhaltene Rohkatalysator enthielt 8,6 Gew.-% Silber und 0,0045 Gew.-% Cäsium.

Der Rohkatalysator wurde mit der folgenden Cäsiumsalzlösung 15 Minuten lang getränkt (Verfahrensschritt c'):

| | |
|---|---|
| Methanol | 95,00 Gew.-% |
| destilliertes Wasser | 4,95 Gew.-% |
| Cäsiumacetat | 0,05 Gew.-%. |

Nach dem Abdekantieren und Trocknen über 30 Minuten bei 100°C (Verfahrensschritt d') wurde ein Silberkatalysator erhalten, der 0,0107 Gew.-% Cäsium und 8,6 Gew.-% Silber enthielt, wobei vom gesamten Cäsium 40 Gew.-% im ersten Imprägnierschritt aufgebracht wurden.

Dieser Katalysator wurde, wie im Beispiel 1, in einem Langzeitversuch von 3 Monaten getestet, wobei das folgende Ergebnis erhalten wurde:

erforderliche Temperatur für

| | |
|---|---|
| 1,3 Vol.-% Ethylenoxid | 196° C |
| Selektivität bei 5 % $C_2H_4$-Umsatz | 81,6 % |
| Selektivität nach 3 Monaten | 81,3 %. |

**Beispiel 7 (Vergleichsbeispiel)**

Bei diesem Vergleichsbeispiel wurde die gesamte Silber- und Cäsiummenge in einem Imprägnierschritt aufgebracht (einstufige gleichzeitige Aufbringung der gesamten Silber- und Promotormenge). Der Träger und die Arbeitsweise entsprachen dem Beispiel 1.

Die Lösung für die Imprägnierung hatte folgende Zusammensetzung:

30,00 g (38,4369 Gew.-%) Silbernitrat
20,00 g (25,6246 Gew.-%) destilliertes Wasser
28,00 g (35,8744 Gew.-%) sekundäres Butylamin
0,05 g ( 0,0641 Gew.-%) Cäsiumnitrat.

Der fertige Katalysator enthielt 8,5 Gew.-% Silber und 0,0130 Gew.-% Cäsium.

Der Test des Katalysators 2 Monate lange mit den in Beispiel 1 beschriebenen Bedingungen zeigte folgendes Ergebnis:

erforderliche Temperatur für

| | |
|---|---|
| 1,3 Vol.-% Ethylenoxid | 193°C |
| Selektivität bei 5 % $C_2H_4$-Umsatz | 80,2 % |
| Selektivität nach 2 Monaten | 80,1 %. |

**Beispiel 8 (Vergleichsbeispiel)**

Bei diesem Vergleichsbeispiel wurde die gesamte Cäsiummenge im zweiten Imprägnierschritt aufgebracht, nachdem im ersten Imprägnierschritt die gesamte Silbermenge aufgebracht worden war. Der Träger und die Arbeitsweise entsprachen dem Beispiel 1.

Die Imprägnierlösung für den ersten Imprägnierschritt hatte die folgende Zusammensetzung:

30,0 g (38,46 Gew.-%) Silbernitrat
20,0 g (25,64 Gew.-%) destilliertes Wasser
28,0 g (35,90 Gew.-%) sekundäres Butylamin.

Der nach der Reduzierung des aufgebrachten Silbernitrates erhaltene Katalysator hatte 8,6 Gew.-% Silber. Die gesamte Cäsiummenge wurde nun mit der folgenden Lösung aufgetragen:

| | |
|---|---|
| Methanol | 95,00 Gew.-% |
| destilliertes Wasser | 4,93 Gew.-% |
| Cäsiumnitrat | 0,07 Gew.-%. |

Der fertige Katalysator enthielt 8,6 Gew.-% Silber und 0,0135 Gew.-% Cäsium.

Der Test dieses Katalysators 1 Monat lang mit den in Beispiel 1 beschriebenen Bedingungen zeigte folgendes Ergebnis:

erforderliche Temperatur für

| | |
|---|---|
| 1,3 Vol.-% Ethylenoxid | 194°C |
| Selektivität bei 5 % $C_2H_4$-Umsatz | 79,8 % |
| Selektivität nach 1 Monat | 79,6 %. |

**Patentansprüche**

1. Silberkatalysator, bestehend aus Silber in einer Menge von 3 bis 20 Gew.-% und Kalium, Rubidium und/oder Cäsium als Promotor in einer Menge von 0,003 bis 0,05 Gew.-% auf einem hitzebeständigen, porösen Trägermaterial, Gewichtsprozente jeweils bezogen auf das Gewicht des Katalysators, wobei das Silber und der Promotor auf das Trägermaterial unter Verwendung von Imprägnierlösungen aufgebracht worden sind und die aufgebrachte Silberverbindung zu metallischem Silber reduziert worden ist, dadurch gekennzeichnet, daß das Aufbringen von Silber und Promotor und die Reduktion nach einer der beiden nachstehenden Methoden durchgeführt worden sind:

a) gleichzeitiges Aufbringen von 55 bis 85 Gew.-% von der gesamten Silbermenge und 15 bis 45 Gew.-% von der gesamten Promotormenge in einer ersten Imprägnierung,
b) Trocken des im Schritt a) erhaltenen Produktes,
c) gleichzeitiges Aufbringen des Restes von der gesamten Silbermenge und Promotormenge auf das im Schritt b) erhaltene Produkt in einer zweiten Imprägnierung und
d) Erhitzen des im Schritt c) erhaltenen Produktes zur Reduzierung der aufgebrachten Silberverbindung zu metallischem Silber;

oder

a') gleichzeitiges oder nacheinanderfolgendes Aufbringen der gesamten Silbermenge und 15 bis 45 Gew.-% von der gesamten Promotormenge in einer beziehungsweise in zwei Imprägnierungen,
b') Erhitzen des im Schritt a') erhaltenen Produktes zur Reduzierung der aufgebrachten Silberverbindung zu metallischem Silber,
c') Aufbringen des Restes von der gesamten Promotormenge auf das in Schritt b') erhaltene Produkt in einer weiteren Imprägnierung und
d') Trocknen des in Schritt c') erhaltenen Produktes, und daß im Falle des Silbers eine im wesentlichen gleichmäßige Verteilung auf der inneren und äußeren Oberfläche des Trägermaterials vorliegt und

im Falle des Promotors die äußere Oberfläche des Trägermaterials eine höhere Konzentration als die innere Oberfläche aufweist.

2. Silberkatalysator nach Anspruch 1, dadurch gekennzeichnet, daß die Silbermenge 7 bis 14 Gew.-% beträgt.

3. Silberkatalysator nach Anspruch 1, dadurch gekennzeichnet, daß die Promotormenge 0,008 bis 0,035 Gew.-% beträgt.

4. Silberkatalysator nach Anspruch 2, dadurch gekennzeichnet, daß der Promotor Cäsium ist.

5. Silberkatalysator nach einem oder mehreren der Ansprüche 1 bis 4, dadurch gekennzeichnet, daß bei der ersten Methode im Schritt a) 60 bis 75 Gew.-% von der gesamten Silbermenge und 25 bis 40 Gew.-% von der gesamten Promotormenge und bei der zweiten Methode im Schritt a') 20 bis 35 Gew.-% von der gesamten Promotormenge aufgebracht worden sind.

6. Verfahren zur Herstellung eines Silberkatalysators nach Anspruch 1, wobei das Silber und der Promotor auf das Trägermaterial unter Verwendung von Imprägnierlösungen aufgebracht werden und die aufgebrachte Silberverbindung zu metallischem Silber reduziert wird, dadurch gekennzeichnet, daß es nach einer der beiden nachstehenden Methoden durchgeführt wird:

a) gleichzeitiges Aufbringen von 55 bis 85 Gew.-% von der gesamten Silbermenge und 15 bis 45 Gew.-% von der gesamten Promotormenge in einer ersten Imprägnierung,
b) Trocken des im Schritt a) erhaltenen Produktes,
c) gleichzeitiges Aufbringen des Restes von der gesamten Silbermenge und Promotormenge auf das im Schritt b) erhaltene Produkt in einer zweiten Imprägnierung und
d) Erhitzen des im Schritt c) erhaltenen Produktes zur Reduzierung der aufgebrachten Silberverbindung zu metallischem Silber;

oder

a') gleichzeitiges oder nacheinanderfolgendes Aufbringen der gesamten Silbermenge und 15 bis 45 Gew.-% von der gesamten Promotormenge in einer beziehungsweise in zwei Imprägnierurgen,
b') Erhitzen des im Schritt a') erhaltenen Produktes zur Reduzierung der aufgebrachten Silberverbindung zu metallischem Silber,
c') Aufbringen des Restes von der gesamten Promotormenge auf das im Schritt b') erhaltene Produkt in einer weiteren Imprägnierung und
d') Trocknen des im Schritt c') erhaltenen Produktes.

7. Verfahren nach Anspruch 6, dadurch gekennzeichnet, daß bei der ersten Methode im Schritt a) 60 bis 75 Gew.-% von der gesamten Silbermenge und 25 bis 40 Gew.-% von der gesamten Promotormenge und bei der zweiten Methode im Schritt a') 20 bis 35 Gew.-% von der gesamten Promotormenge aufgebracht werden.

8. Verwendung des Silberkatalysators nach Anspruch 1 zur Herstellung von Ethylenoxid durch direkte Oxidation von Ethylen mit molekularem Sauerstoff bei einer Temperatur von 200 bis 270°C unter Anwendung eines Katalysatorfestbettes.

## Claims

1. A silver catalyst which consists of silver in an amount of 3 to 20 % by weight and potassium, rubidium and/or cesium as a promoter in an amount of 0.003 to 0.05 % by weight on a heat-resistant porous support material, all the weight percentages being relative to the weight of the catalyst, the silver and promoter having been applied to the support material in the form of impregnating solutions and the applied silver compound having been reduced to metallic silver, characterised in that the application of silver and promoter and the reduction have been carried out by one of the two following methods:

a) simultaneous application of 55 to 85 % by weight of the total amount of silver and 15 to 45 % by weight of the total amount of promoter in a first impregnating step,
b) drying of the product obtained in step a),
c) simultaneous application of the resf of the total amount of silver and promoter in a second impregnating step to the product obtained in step b), and
d) heating of the product obtained in step c) to reduce the applied silver compound to metallic silver,

or

a') simultaneous or successive application of the total amount of silver and 15 to 45 % by weight of the total amount of promoter in, respectively, one or two impregnating steps,
b') heating of the product obtained in step a') to reduce the applied silver compound to metallic silver,

c') application of the rest of the total amount of promoter in a further impregnating step to the product obtained in step b'), and

d') drying of the product obtained in step c'), and that the silver is essentially uniformly distributed over the inner and outer surfaces of the support material, and as to the promoter the outer surfaces of the supported material have a higher concentration than the inner surfaces.

2. The silver catalyst claimed in claim 1, wherein the amount of silver is 7 to 14 % by weight.

3. The silver catalyst claimed in claim 1, wherein the amount of promoter is 0.08 to 0.035 % by weight.

4. The silver catalyst claimed in claim 1, wherein the promoter is cesium.

5. The silver catalyst claimed in one or more of the claims 1 to 4, wherein in step a) of the first method 60 to 75 % by weight of the total amount of silver and 25 to 40 % by weight of the total amount of promoter have been applied and in step a') of the second method 20 to 35 % by weight of the total amount of promoter.

6. A process for preparing a silver catalyst as claimed in claim 1 by applying the silver and the promoter to the support material in the form of impregnating solutions and reducing the applied silver compound to metallic silver, characterised in that it is carried out by one of the two following methods:

a) simultaneous application of 55 to 85 % by weight of the total amount of silver and 15 to 45 % by weight of the total amount of promoter in a first impregnating step,

b) drying of the product obtained in step a),

c) simultaneous application of the rest of the total amount of silver and promoter in a second impregnating step to the product obtained in step b), and

d) heating of the product obtained in step c) to reduce the applied silver compound to metallic silver,

or

a') simultaneous or successive application of the total amount of silver and 15 to 45 % by weight of the total amount of promoter in, respectively, one or two impregnating steps,

b') heating of the product obtained in step a') to reduce the applied silver compound to metallic silver,

c') application of the rest of the total amount of promoter in a further impregnating step to the product obtained in step b'), and

d') drying of the product obtained in step c').

7. The process claimed in claim 6, wherein in step a) of the first method 60 to 75 % by weight of the total amount of silver and 25 to 40 % by weight of the total amount of promoter are applied and in step a') of the second method 20 to 35 % by weight of the total amount of promoter.

8. The use of the silver catalyst claimed in claim 1 for preparing ethylene oxide by direct oxidation of ethylene with molecular oxygen at a temperature of 200 to 270°C over a fixed silver catalyst bed.

## Revendications

1. Catalyseur à base d'argent, constitué d'argent en une quantité de 3 à 20 % en poids et, comme promoteur, de potassium, de rubidium et/ou de césium en une quantité de 0,003 à 0,05 % en poids, à chaque fois par rapport au poids du catalyseur, sur une matière support poreuse résistant à la chaleur, l'argent et le promoteur ayant été appliqués sur la matière support au moyen de solutions d'imprégnation et le composé de l'argent appliqué ayant été réduit en argent métallique, catalyseur caractérisé en ce que l'application de l'argent et du promoteur et la réduction ont été effectuées par l'une des deux méthodes suivantes:

a) on applique en même temps de 55 à 85 % en poids de la quantité totale d'argent et de 15 à 45 % en poids de la quantité totale du promoteur, dans une première imprégnation,

b) on sèche le produit obtenu à l'étape a),

c) on applique en même temps, dans une seconde imprégnation, le reste de la quantité totale d'argent et le reste de la quantité totale du promoteur sur le produit obtenu à l'étape b), et

d) on chaffe le produit obtenu à l'étape c) pour réduire en argent métallique le composé argentique qui a été appliqué,

ou

a') on applique, en même temps ou successivement, la quantité totale d'argent et de 15 à 45 % en poids de la quantité totale du promoteur, en une ou en deux imprégnations,

b') on chaffe le produit obtenu à l'étape a') pour réduire en argent métallique le composé argentique qui a été appliqué,

c') on applique le reste de la quantité totale du promoteur sur le produit obtenu à l'étape b'), dans une autre étape d'imprégnation, et

d') on sèche le produit obtenu à l'étape c'),

et en ce que l'argent est réparti d'une façon pratiquement homogène sur la surface intérieure et sur la surface extérieure de la matière support, tandis que la surface extérieure de la matière support a une concentration plus élevée en promoteur que la surface intérieure.

2. Catalyseur à base d'argent selon la revendication 1, caractérisé en ce que la quantité d'argent est comprise entre 7 et 14 % en poids.

3. Catalyseur à base d'argent selon la revendication 1, caractérisé en ce que la quantité du promoteur est comprise entre 0,008 et 0,035 % en poids.

4. Catalyseur à base d'argent selon la revendication 1, caractérisé en ce que le promoteur est le césium.

5. Catalyseur à base d'argent selon l'une quelconque des revendications 1 à 4, caractérisé en ce que, dans la première méthode, on a appliqué, à l'étape a), de 60 à 75 % en poids de la quantité totale d'argent et de 25 à 40 % en poids de la quantité totale du promoteur et, dans la seconde méthode, on a appliqué, à l'étape a'), de 20 à 35 % en poids de la quantité totale du promoteur.

6. Procédé de préparation d'un catalyseur à base d'argent selon la revendication 1 dans lequel l'argent et le promoteur sont appliqués sur la matière support au moyen de solutions d'imprégnation et le composé argentique appliqué est réduit en argent métallique, procédé caractérisé en ce qu'il est exécuté selon l'une des deux méthodes suivantes :

a) on applique en même temps de 55 à 85 % en poids de la quantité totale d'argent et de 15 à 45 % en poids de la quantité totale du promoteur, dans une première imprégnation,
b) on sèche le produit obtenu à l'étape a),
c) on applique en même temps, dans une seconde imprégnation, le reste de la quantité totale d'argent et le reste de la quantité totale du promoteur sur le produit obtenu à l'étape b), et
d) on chauffe le produit obtenu à l'étape c) pour réduire en argent métallique le composé argentique qui a été appliqué,

ou

a') on applique, en même temps ou successivement, la quantité totale d'argent et de 15 à 45 % en poids de la quantité totale du promoteur, en une ou en deux imprégnations,
b') on chauffe le produit obtenu à l'étape a') pour réduire en argent métallique le composé argentique qui a été appliqué,
c') on applique le reste de la quantité totale du promoteur sur le produit obtenu à l'étape b'), dans une autre étape d'imprégnation, et
d') on séche le produit obtenu à l'étape c').

7. Procédé selon la revendication 6 caractérisé en ce que, dans la première méthode, on applique, à l'étape a), de 60 à 75 % en poids de la quantité totale d'argent et de 25 à 40 % en poids de la quantité totale du promoteur, et, dans la deuxième méthode, on applique, à l'étape a'), de 20 à 55 % en poids de la quantité totale du promoteur.

8. Application du catalyseur à base d'argent selon la revendication 1 à la préparation de l'oxyde d'éthylène par oxydation directe de l'éthylène par l'oxygène moléculaire, à une température de 200 à 270°C, le catalyseur étant utilisé sous la forme d'un lit fixe.